# EUROPEAN PATENT APPLICATION

(11) **EP 4 613 281 A1**
(43) Date of publication of application: **10.09.2025**
(21) Application number: 23885977.1
(22) Date of filing: 22.08.2023
(51) Int. Cl.: A61K 36/87, A61K 31/343, A61P 25/28, A23L 33/10, A23L 33/105, A23K 10/30, A23K 20/121

(54) **COMPOSITION FOR PREVENTING, AMELIORATING, OR TREATING ALZHEIMER'S DISEASE CONTAINING AS ACTIVE INGREDIENT VITIS VINIFERA STEM EXTRACT OR COMPOUND ISOLATED THEREFROM**

(30) Priority: 31.10.2022 KR 20220141967
(71) Applicant: Korea Institute of Oriental Medicine, Daejeon 34054 (KR); Gyeonggido Business & Science Accelerator, Suwon-si, Gyeonggi-do 16229 (KR)
(72) Inventor: CHOI, Jang-Gi, Daegu 41088 (KR); KWON, Eun-Bin, Daegu 41062 (KR); KIM, Young Soo, Hwaseong-si Gyeonggi-do 18477 (KR); LI, Wei, Seoul 01907 (KR); KIM, Buyun, Daegu 41025 (KR); GO, Younghoon, Gyeongsan-si Gyeongsangbuk-do 38699 (KR); CHOI, Chunwhan, Yongin-si Gyeonggi-do 16854 (KR)
(74) Representative: Ipsilon
(86) International application number: PCT/KR2023/012385
(87) International publication number: WO 2024/096280

(57) **Abstract**

The present invention relates to a composition for preventing, ameliorating, or treating Alzheimer's disease comprising grapevine stem extract or a compound isolated therefrom as an effective component. Specifically, as the grapevine stem extract and vitisin A as an effective component of the present invention exhibit an excellent effect of inhibiting infection with the herpes simplex virus and inhibiting Alzheimer-causing factors in the brain tissue of animal models infected with the herpes simplex virus, the composition of the present invention can be advantageously used as an agent for treating Alzheimer's disease or a functional health food or an animal feed additive for preventing or ameliorating Alzheimer's disease.

## Description

### TITLE OF INVENTION

Composition for preventing, ameliorating, or treating Alzheimer's disease comprising grapevine stem extract or compound isolated therefrom as effective component

### TECHNICAL FIELD

The present invention relates to a composition for preventing, ameliorating, or treating Alzheimer's disease comprising grapevine stem extract or a compound isolated therefrom as an effective component.

This work was conducted with support from the Personal Basic Research Project of the Ministry of Science and ICT, South Korea (Project Number: 2021R1A2C2094436).

### BACKGROUND ART

Alzheimer's disease (AD) is a disease in which brain neurons (i.e., nerve cells) involved in learning and memory die, resulting in loss of memory and gradual decline in thinking ability, including calculation ability, language ability, spatiotemporal understanding, and judgment.

Alzheimer's disease is one of the chronic neurodegenerative diseases and accounts for 60 to 70% of the causes of dementia. The cause of Alzheimer's disease is not well known, but death of nerve cells is commonly observed in the limbic pathway, which controls memory. The likely cause of the gradual loss of cognitive function, a key characteristic of AD patients, appears to be Aβ (amyloid beta) accumulated at abnormally high level.

Current studies on Alzheimer's disease is focused on controlling the accumulation of abnormal proteins, and although many therapeutic agents that inhibit Aβ aggregation have been developed and clinically tested, none have shown a special therapeutic effect.

Meanwhile, Herpes is a herpes simplex virus that causes blisters on the skin, lips, eyes, and genitals. The Greek word 'herpin', the origin of the word herpes, means 'latent'. Herpes simplex virus (HSV), the causative agent of herpes simplex, is infected during childhood and remains latent for a long period of time. Everyone has suffered from recurring blisters around the mouth or eyes when they are tired or stressed. This happens because HSV, which was latent in the body, becomes activated and relapses. It is characterized by blistering lesions that appear acutely at the border between the skin and mucous membranes, but its appearance makes it feel embarrassing. The virus that causes the incredibly painful shingles is also one type of the herpes. It hides in the ganglia and, in accordance with aging and decreased immunity, reappears in the form of shingles. HSV is a very common virus in humans, with research showing that 95% of adults are infected with HSV.

Recently, the theory that Alzheimer's dementia is caused by a virus has been receiving attention, and it has been additionally observed that HSV-1 infection is actually related to Alzheimer's disease. In October 2014, Dr. Hugo Löbheim's laboratory at Umeå University School of Medicine in Sweden reported that people who were infected with HSV-1 had a higher risk of dementia than people who were not infected. In particular, in the case of elderly people over 60 years of age, if the period of herpes simplex symptoms appearing for more than 6.6 years, the risk of having Alzheimer's dementia increases by 2.3 times, and if the number of herpes simplex infections is high, the possibility of developing Alzheimer's dementia also increases. Therefore, reactivation and spread of HSV-1 infection have been suggested to be responsible for cognitive decline, which may be particularly associated with Alzheimer's disease.

Meanwhile, grapes *(Vitis vinifera)* are deciduous vines belonging to the grape family and are one of the most cultivated fruits, accounting for about 30% of the world's fruit production. Grapes contain various organic acids such as glucose, fructose, sucrose, citric acid, tartaric acid, acetic acid, and malic acid, as well as vitamins A, B, and C, and the skin of grapes contains well-known anthocyanin components such as peonidin, delphinidin, malvidin, and petunidin. However, research on grapevine stem extracts or stem bark extracts is insufficient.

Grapevine is pruned every year, resulting in cut vine stems, which are discarded by farms because they are not economically viable. Therefore, if functional foods and high value-added products can be developed through research on the functionality of discarded grapevine stems or grapevine stem bark, they can be used as a new source of income for farmers.

As prior art related to grapes, Korean Patent Registration No. 2025572 discloses a composition for preventing, ameliorating, or treating metabolic diseases containing a mixture of extracts of the leaves of a date plum and grape stem as an effective component, and Korean Patent Application Publication No. 2020-0004134 discloses a composition for improving skin containing grape callus culture fluid as an effective component. Furthermore, Korean Patent Application Publication No. 2018-0136201 discloses a composition for preventing or treating noroviral diarrhea containing grape root extract or a fraction thereof as an effective component. However, there has been no disclosure regarding a composition for preventing, ameliorating, or treating Alzheimer's disease comprising grapevine stem extract or a compound isolated therefrom as an effective component.

### DETAILED DESCRIPTION OF INVENTION

### TECHNICAL PROBLEMS TO BE SOLVED

The present invention is devised in view of the need described above, and provided by the present invention is a composition for preventing, ameliorating, or treating Alzheimer's disease containing grapevine stem extract or vitisin A isolated therefrom as an effective component. Specifically, it is found that the grapevine stem extract or vitisin A inhibits infection with herpes simplex virus and inhibits the production of amyloid beta, an Alzheimer-causing factor, and phosphorylation of tau protein in the brain tissue of an animal model infected with herpes simplex virus. The present invention is completed accordingly.

### TECHNICAL MEANS FOR SOLVING THE PROBLEMS

To solve the problems that are described in the above, the present invention provides a pharmaceutical composition for preventing or treating Alzheimer's disease comprising grapevine stem extract or vitisin A as an effective component.

The present invention further provides a functional health food composition for preventing or ameliorating Alzheimer's disease comprising grapevine stem extract or vitisin A as an effective component.

The present invention still further provides an animal feed additive for preventing or ameliorating Alzheimer's disease comprising grapevine stem extract or vitisin A as an effective component.

### ADVANTAGEOUS EFFECT OF INVENTION

The present invention relates to a composition for preventing, ameliorating, or treating Alzheimer's disease comprising grapevine stem extract or a compound isolated therefrom as an effective component. It is found that grapevine stem (i.e., fruiting stem) extract, which is an effective component of the present invention, inhibits intracellular infection of the HSV-1 virus better than the grapevine main stem bark extract, and, as a result of determining the antiviral activity of the compound isolated from the grapevine stem extract against the HSV-1 virus, it is shown that the intracellular infection inhibitory effect of vitisin A and vitisin B is more significant than that of other isolated compounds (i.e.., ε-viniferin, caraphenol A, betuin, cis-vitisin). A, amphelopsin A, lupeol, malivatol A, vinifeether B, lufenone, and resveratrol).

Furthermore, in an animal model of HSV-1 infection, the grapevine stem extract reduced mortality due to viral infection and suppressed weight loss.

Still furthermore, administration of the grapevine stem extract or vitisin A reduced viral protein expression in brain tissue of HSV-1 infected animal model, and they were found to be highly effective in suppressing the production of amyloid beta, an Alzheimer-causing factor, and the phosphorylation of tau protein.

### BRIEF DESCRIPTION OF THE DRAWINGS

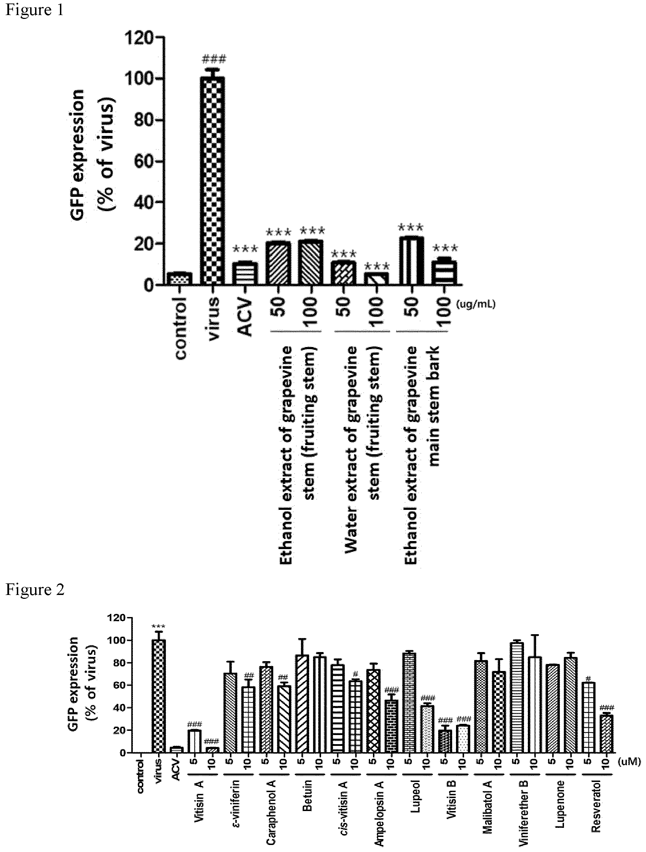

Figure 1 shows the results of determining the effect of inhibiting herpes simplex virus infection in Vero cells by treatment with an ethanol extract of the grapevine stem (i.e., fruiting stem), a water extract of the grapevine stem (i.e., fruiting stem), both the extract of the present invention, or an extract of the grapevine main stem bark. Control is a group not treated with any virus or sample, Virus is a group in which only the herpes simplex virus was treated without any sample treatment, and ACV is a positive control group treated with acyclovir. ### indicates that, compared to Control, the degree of viral infection increased statistically significantly in the virus group, p<0.001. *** indicates a statistically significant decrease in the degree of virus infection in the sample treatment group compared to the virus group, p<0.001.
Figure 2 shows the results of determining the effect of inhibiting herpes simplex virus infection in Vero cells by treatment with a compound contained in the grapevine stem extract of the present invention. Control is a group not treated with any virus or sample, Virus is a group in which only the herpes simplex virus was treated without any sample treatment, and ACV is a positive control group treated with acyclovir. *** indicates that, compared to the control, the degree of viral infection increased statistically significantly in the virus group, p<0.001. #, ##, and ### indicate a statistically significant decrease in the degree of virus infection in the sample treatment group compared to the virus group, in which # p<0.05, ## p<0.01, and ### p<0.001.

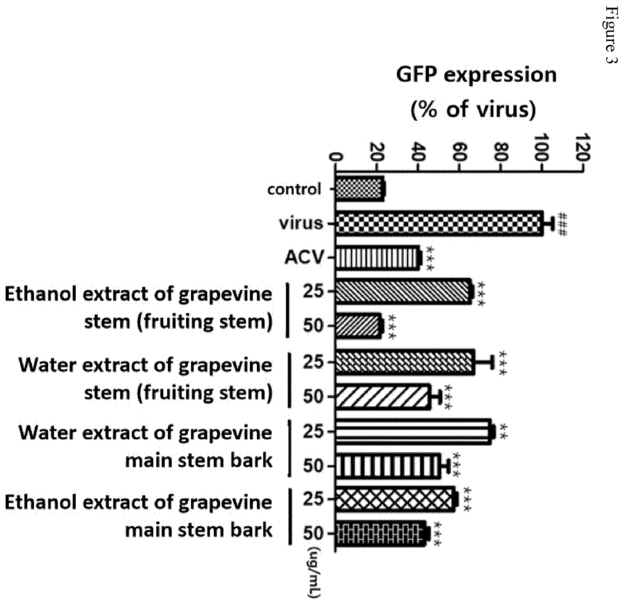

Figure 3 shows the results of determining the effect of inhibiting herpes simplex virus infection in SK-N-SH cells by treatment with an ethanol extract of the grapevine stem (i.e., fruiting stem), a water extract of the grapevine stem (i.e., fruiting stem), both the extract of the present invention, or an extract of the grapevine main stem bark. Control is a group not treated with any virus or sample, Virus is a group in which only the herpes simplex virus was treated without any sample treatment, and ACV is a positive control group treated with acyclovir. ### indicates that, compared to Control, the degree of viral infection increased statistically significantly in the virus group, p<0.001. ** and *** indicate a statistically significant decrease in the degree of virus infection in the sample treatment group compared to the virus group, in which ** p<0.01, and *** p<0.001.

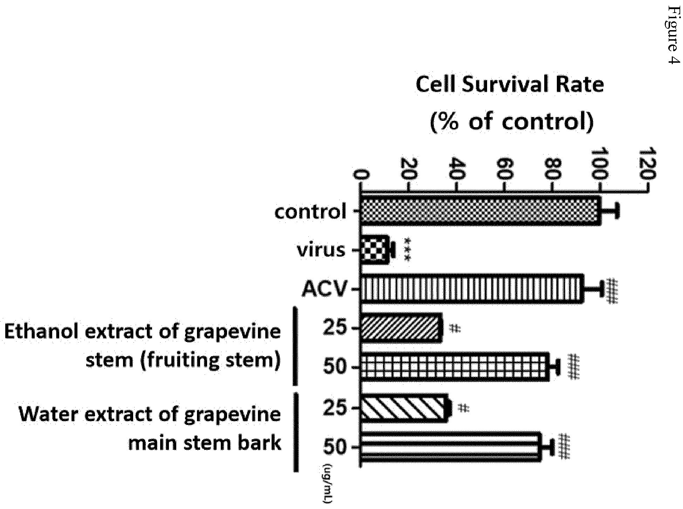

Figure 4 shows the results of determining a change in the cell survival rate after herpes simplex virus infection in U373-MG cells followed by a treatment with an ethanol extract of the grapevine stem (i.e., fruiting stem) of the present invention or an extract of the grapevine main stem bark. Control is a group not treated with any virus or sample, Virus is a group in which only the herpes simplex virus was treated without any sample treatment, and ACV is a positive control group treated with acyclovir. *** indicates that, compared to Control, the cell survival rate decreased statistically significantly in the virus group, p<0.001. # and ### indicate a statistically significant increase in the cell survival rate in the sample treatment group compared to the virus group, in which # p<0.05, and ### p<0.001.

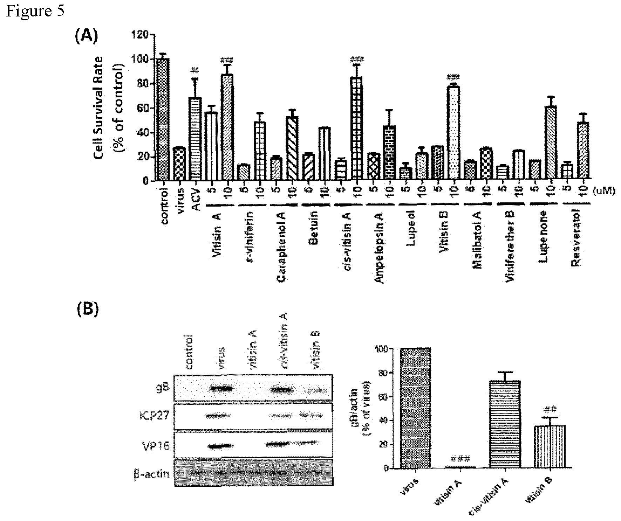

Figure 5 shows the results of determining (A) a change in the cell survival rate and (B) expression level of the viral proteins after herpes simplex virus infection in U373-MG cells followed by a treatment with a compound contained in the grapevine stem extract of the present invention. Control is a group not treated with any virus or sample, Virus is a group in which only the herpes simplex virus was treated without any sample treatment, and ACV is a positive control group treated with acyclovir. ## and ### indicate a statistically significant increase in the cell survival rate in the sample treatment group compared to the virus group, or a statistically significant decrease in the expression of the gB viral proteins in the sample treatment group compared to the virus group, in which ## p<0.01, and ### p<0.001.

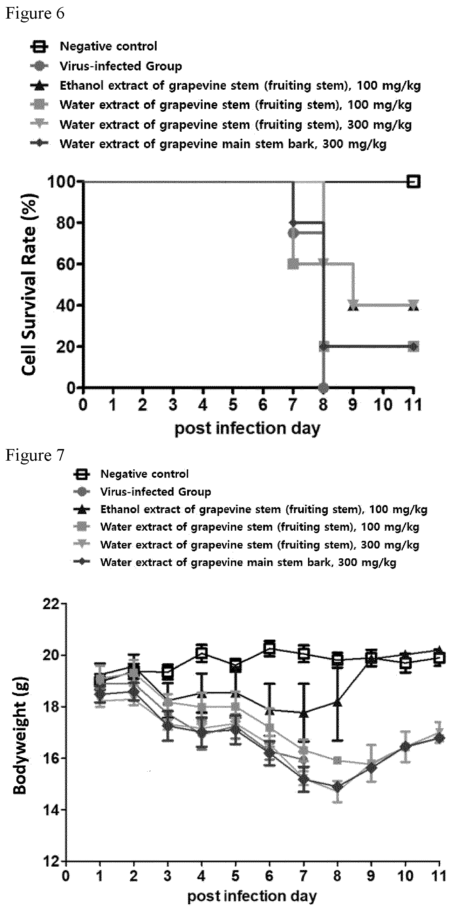

Figure 6 shows the results of determining the survival rate after administration of grapevine stem(fruiting stem) extract or grapevine main stem bark extract in a virus infection animal model.
Figure 7 shows the results of determining a change in body weight caused by administration of grapevine stem(fruiting stem) extract or grapevine main stem bark extract in a virus infection animal model.

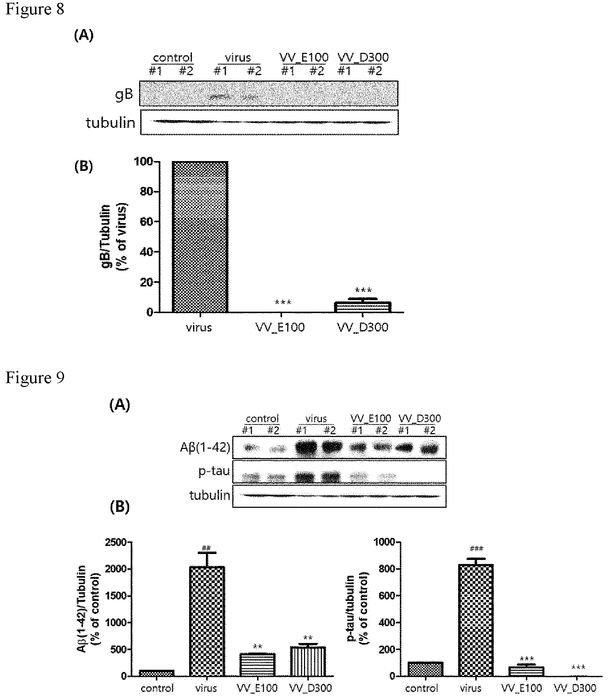

Figure 8 shows the results of determining the expression of gB viral protein after administration of an ethanol extract of the grapevine stem (i.e., fruiting stem) or a water extract of the grapevine stem (i.e., fruiting stem) in brain tissue of a virus infection animal model. Figure 8(A) show the result of Western Blot analysis and Figure 8(B) shows its quantitative result. Control is a group not treated with any virus or sample, and Virus is a group in which only the herpes simplex virus was treated without any sample treatment. VV-E100 is a group that is infected with virus and administered with 100 mg/kg ethanol extract of the grapevine fruiting stem, and VV-D300 is a group that is infected with virus and administered with 300 mg/kg of water extract of grapevine fruiting stem. *** indicates a statistically significant decrease in gB protein expression in the sample administration group compared to the virus group, p<0.001.
Figure 9 shows the results of determining a change in the expression of amyloid beta (Aβ1-42) protein, which is an Alzheimer-related factor, and also degree of the phosphorylation of tau protein (i.e., p-tau) after administration of an ethanol extract of the grapevine stem (i.e., fruiting stem) or a water extract of the grapevine stem (i.e., fruiting stem) in brain tissue of a virus infection animal model. Figure 9(A) show the result of Western Blot analysis and Figure 9(B) shows their quantitative results. Control is a group not treated with any virus or sample, and Virus is a group in which only the herpes simplex virus was treated without any sample treatment. VV-E100 is a group that is infected with virus and then administered with 100 mg/kg ethanol extract of the grapevine fruiting stem, and VV-D300 is a group that is infected with virus and then administered with 300 mg/kg of water extract of grapevine fruiting stem. ## and ### indicate a statistically significant increase in the expression of amyloid beta (Aβ1-42) protein and also in the degree of the phosphorylation of tau protein (i.e., p-tau) in the virus group compared to Control, in which ## p<0.01, and ### p<0.001. ** and *** indicate a statistically significant decrease in the expression of amyloid beta (Aβ1-42) protein and also in the degree of the phosphorylation of tau protein (i.e., p-tau) in the sample administration group compared to the virus group, in which ** p<0.01, and *** p<0.001.

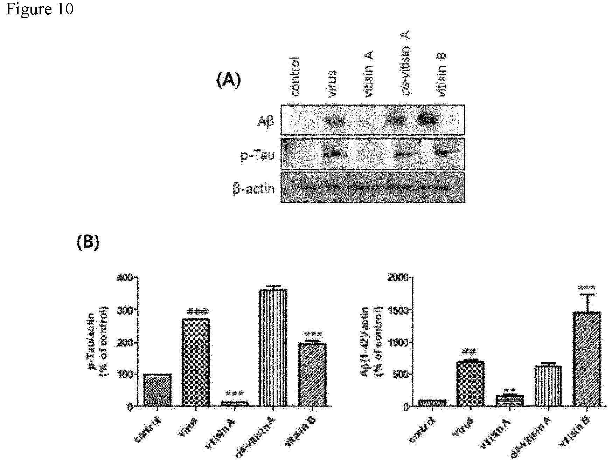

Figure 10 shows the results of determining a change in the expression of amyloid beta (Aβ1-42) protein, which is an Alzheimer-related factor, and also degree of the phosphorylation of tau protein (i.e., p-tau) after administration of a compound isolated from the grapevine stem in a virus infection cell model. Figure 10(A) show the result of Western Blot analysis and Figure 10(B) shows their quantitative results. Control is a group not treated with any virus or sample, and Virus is a group in which only the herpes simplex virus was treated without any sample treatment. Vitisin A is a group that is infected with virus and then administered with 10 µM vitisin A, cis-Vitisin A is a group that is infected with virus and then administered with 10 µM cis-vitisin A, and Vitisin B is a group that is infected with virus and then administered with 10 µM vitisin-B. ## and ### indicate a statistically significant increase in the expression of amyloid beta (Aβ1-42) protein and also in the degree of the phosphorylation of tau protein (i.e., p-tau) in the virus group compared to Control, in which ## p<0.01, and ### p<0.001. ** and *** indicate a statistically significant decrease in the expression of amyloid beta (Aβ1-42) protein and also in the degree of the phosphorylation of tau protein (i.e., p-tau) in the sample administration group compared to the virus group, in which ** p<0.01, and *** p<0.001.

### BEST EMBODIMENT(S) FOR CARRYING OUT INVENTION

To achieve the object that is described in the above, the present invention provides a pharmaceutical composition for preventing or treating Alzheimer's disease comprising grapevine stem extract or vitisin A as an effective component.

The Alzheimer's disease may be Alzheimer's dementia caused by a herpes virus infection, and the herpes virus is preferably herpes simplex virus type 1 (HSV-1), but is not limited thereto.

The vitisin A may be isolated from grapevine stem extract, but is not limited thereto.

The grapevine stem extract of the present invention may be an extract of any one selected from grapevine main stem, main branch, fruiting stem, side branch, fruiting branch, bark of the main stem, bark of the main branch, bark of the fruiting stem, bark of the side branch, and bark of the fruiting branch, and is preferably an extract of the fruiting stem to which the fruiting branch, which is a branch bearing fruit, is attached, but it is not particularly limited.

The fruiting stem used in the present invention is a part cut off by pruning.

The extraction solvent for the extract may be water, C₁ to C₄ lower alcohol, or a mixture thereof, and the extraction is preferably made by using ethanol, but is not limited thereto.

The vitisin A of the present invention is a compound of the following Chemical Formula 1.

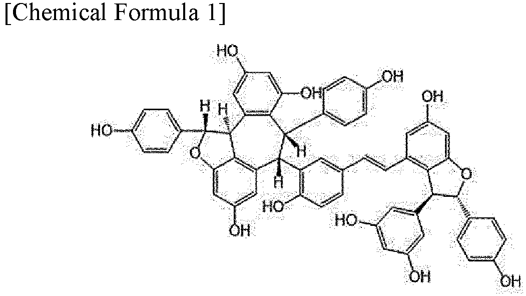

In one embodiment of the present invention, the grapevine stem extract or vitisin A has the characteristic of inhibiting the production of amyloid beta and the phosphorylation of tau protein.

The pharmaceutical composition according to the present invention can be formulated into oral forms such as capsules, powders, granules, tablets, suspensions, emulsions, syrups, and aerosols, as well as external preparations, suppositories, and sterile injectable solutions using conventional methods.

The pharmaceutical composition according to the present invention may further include pharmaceutically acceptable carriers, excipients, or diluents.

The carriers, excipients, and diluents that may be included in the pharmaceutical composition of the present invention include various compounds or mixtures such as lactose, dextrose, sucrose, sorbitol, mannitol, xylitol, erythritol, maltitol, starch, acacia gum, alginate, gelatin, calcium phosphate, calcium silicate, cellulose, methyl cellulose, microcrystalline cellulose, polyvinylpyrrolidone, water, methyl hydroxybenzoate, propyl hydroxybenzoate, talc, magnesium stearate, and mineral oil.

When formulating, the pharmaceutical composition is prepared using common diluents or excipients such as fillers, bulking agents, binders, wetting agents, disintegrants, and surfactants. Solid dosage forms for oral administration include tablets, pills, powders, granules, and capsules. These solid dosage forms are typically prepared by mixing at least one excipient, such as starch, calcium carbonate, sucrose, lactose, or gelatin with the pharmaceutical composition of the present invention.

Additionally, lubricants like magnesium stearate and talc may also be used alongside simple excipients. For liquid dosage forms for oral administration, suspensions, solutions, emulsions, and syrups are usable. Commonly used diluents such as water and liquid paraffin, as well as various excipients like wetting agents, sweeteners, flavoring agents, and preservatives, may be included. For parenteral dosage forms, preparations include sterile aqueous solutions, non-aqueous solutions, suspensions, emulsions, freeze-dried formulations, and suppositories. Non-aqueous solutions and suspensions can use propylene glycol, polyethylene glycol, plant oils like olive oil, or injectable esters like ethyl oleate. As suppository bases, materials such as Witepsol, Macrogol, Tween 61, cocoa butter, laurin fat, and glycerogelatin can be used.

The appropriate dosage of the pharmaceutical composition of the present invention may vary depending on various factors such as the formulation method, administration route, patient's age, weight, gender, pathological condition, diet, administration time, administration route, excretion rate, and responsiveness.

The pharmaceutical composition of the present invention can be administered either orally or parenterally. In the case of parenteral administration, it can be administered topically on the skin, by intravenous injection, subcutaneous injection, intramuscular injection, intraperitoneal injection, or transdermal delivery.

The present invention further provides a functional health food composition for preventing or ameliorating Alzheimer's disease comprising grapevine stem extract or vitisin A as an effective component.

The Alzheimer's disease may be Alzheimer's dementia caused by a herpes virus infection, and the herpes virus is preferably herpes simplex virus type 1 (HSV-1), but is not limited thereto.

The composition may be prepared in any one of formulations selected from powder, granule, pill, tablet, capsule, candy, syrup, and beverage, but it is not limited to them.

When the functional health food composition of the present invention is used as a food additive, it may be added as is or used together with other foods or food ingredients, and it can be used appropriately according to conventional methods. The amount of effective component can be adjusted depending on its intended purpose (prevention or amelioration). Generally, when manufacturing food or beverages, the functional health food composition of the present invention is added in an amount not exceeding 15 parts by weight of the total ingredients, and preferably not exceeding 10 parts by weight. However, for long-term consumption for health purposes, the amount may be below the specified range, and since there are no safety concerns, the effective components can be used in amounts exceeding the specified range.

There are no specific limitations on the types of functional health foods. Examples of foods to which the functional health food composition can be added include meat, sausages, bread, chocolates, candies, snacks, confectioneries, pizza, ramen, noodles, gum, dairy products such as ice cream, various soups, beverages, tea drinks, alcoholic beverages, and vitamin supplements, as well as any health foods in the general sense.

Additionally, the functional health food composition of the present invention can be manufactured as a food, particularly as a functional food. The functional food of the present invention includes ingredients commonly added during food manufacturing, such as proteins, carbohydrates, fats, nutrients, and seasonings. For example, when manufactured as a drink, natural carbohydrates or flavoring agents may be included as additional ingredients, in addition to the effective components. The natural carbohydrates are preferably monosaccharides (e.g., glucose, fructose), disaccharides (e.g., maltose, sucrose), oligosaccharides, polysaccharides (e.g., dextrin, cyclodextrin), or sugar alcohols (e.g., xylitol, sorbitol, erythritol). The flavoring agents can include natural flavoring agents (e.g., thaumatin, stevia extract) and synthetic flavoring agents (e.g., saccharin, aspartame).

In addition to the functional health food composition, various supplements, vitamins, electrolytes, flavoring agents, colorants, pectinic acid and its salts, alginic acid and its salts, organic acids, protective colloid thickeners, pH adjusters, stabilizers, preservatives, glycerin, alcohol, carbonation agents used in carbonated beverages, and the like may also be included. The ratio of these additional ingredients is not critically important, but it is generally selected within the range of 0.01 to 0.1 part by weight with respect to 100 parts by weight of the functional health food composition of the present invention.

The present invention still further provides an animal feed additive for preventing or ameliorating Alzheimer's disease comprising grapevine stem extract or vitisin A as an effective component.

The animal feed additive of the present invention corresponds to an auxiliary feed as defined in the Feed Management Act. In the present invention, the term 'feed' refers to any natural or artificial food, such as a formulated diet, a single meal, or the components of such meals, that animals eat, ingest, and digest. The types of feed are not particularly limited, and any feed commonly used in the relevant technical field can be used. Non-limiting examples of the feed include plant-based feeds such as grains, tubers, food processing byproducts, seaweeds, fiber sources, pharmaceutical byproducts, oils and fats, starches, or grain byproducts; and proteins, inorganic materials, oils and fats, minerals, single-cell proteins, and animal-based feeds such as animal plankton or food. These can be used either alone or in combination of two or more of them.

Hereinbelow, the present invention is explained in greater detail in view of Preparation examples and Examples. However, the following Preparation examples and Examples are given only for more specific explanation of the present invention and it is evident to a person who has common knowledge in the pertinent art that the scope of the present invention is not limited by them.

### EXAMPLES

### Preparation example 1. Preparation of grapevine stem extract

Grapes were first harvested in vineyards in Yuseong, Daejeon (2008) and Daegu, Gyeongbuk (2021) regions in South Korea. Then, fruiting stems (i.e., part from which a fruiting branch, which is a branch bearing fruit, is growing, bearing mother branch) were cut through pruning (i.e., branch trimming), and used as the grapevine (*Vitis vinifera*) stem of the present invention.

Ethanol (100%, 10 times (v/v)) was added to 10 kg of the above-obtained grapevine stem which had been left cut as is, refluxed and cooled (75°C) in a heating mantle for 7 days, and then extraction was carried out twice. Thereafter, a filter paper was placed in a Buchner funnel for filtration and the filtered solution obtained under reduced pressure was concentrated using a vacuum concentrator to have 708.8 g of an ethanol extract of the grapevine stem.

In addition, 2 L of distilled water were added to 750 g of the grapevine stem which had been left cut as is, and extraction was carried out in a water bath (100°C) for 2 hours. Then, a filter paper was placed in a Buchner funnel for filtration and the filtered solution obtained under reduced pressure was concentrated using a freeze dryer to have a water extract of the grapevine stem (fruiting stem) (31.83 g).

In addition, 750 g of grapevine main stem bark (trunk, a branch of the tree's central axis rising from the root) were extracted in the same manner as above to yield an ethanol extract of the grapevine main stem bark (2.9 g); or a water extract of the grapevine main stem bark (2.86 g).

### Preparation example 2. Isolation of compound from grapevine stem extract

The ethanol extract of grapevine stem from Preparation example 1 was suspended in water and sequentially distributed with equal volumes of CH₂Cl₂, EtOAc, and n-BuOH. Using silica gel (230 to 400 mesh; 250 g) chromatography, three fractions (1 to 3) were suspended in an organic solvent according to the characteristics of each fraction to create a column of 9 : 1 size, then dissolved in the initial mobile phase and loaded on the column. After flowing 2 to 8 L of the mobile phase solvent, the fractions containing pure compounds were determined by TLC, combined, and concentrated to obtain the fraction.

The fraction was heated and dissolved in MeOH solvent, left at room temperature to precipitate crystals, filtered, and recrystallized to obtain a pure compound. The separated compounds were dried under reduced pressure at 40°C for 2 hours, then lupenone, lupeol, and betuin were isolated from fraction 1, vitisin A, cis-vitisin A, vitisin B, malibatol A, and ε-viniferin was isolated were isolated from fraction 2, and ampelopsin A, viniferether B, caraphenol A, and resveratrol were isolated from fraction 3.

The primary planar structures of the isolated compounds were determined through 1D (¹H and ¹³C) NMR analysis, and the secondary three-dimensional structures were determined through 2D NMR (COZY, HSQC, HMBC, and NOESY) analysis.

Additionally, the molecular weight and mass of the compounds were determined through mass spectrometry (MS/MS).

Vitisin A: Brown amorphous powder; [α]20/D = +209.2° (c 0.15, MeOH) HRFABMS: 907.2757 [M+H]⁺; C₅₆H₄₃O₁₂), calcd. 907.2755

¹H and ¹³C NMR values of vitisin A are described in the following Table 1.

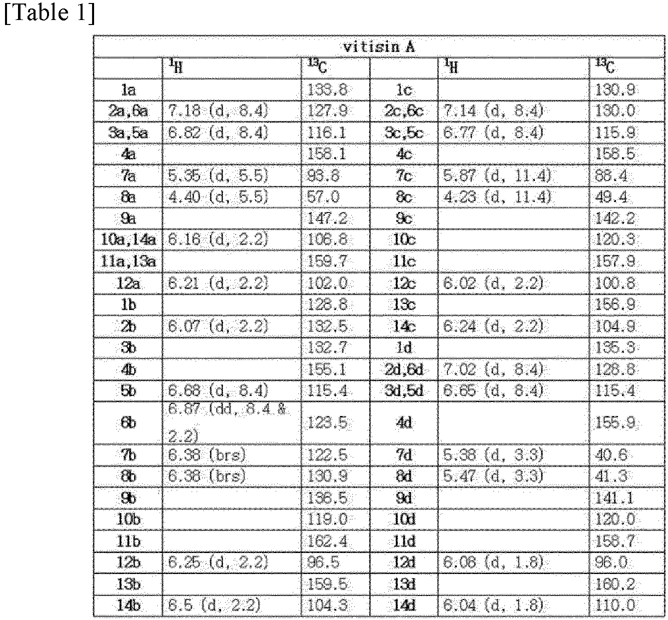

### Example 1. Antiviral effect of extract of grapevine stem and extract of grapevine main stem bark in Vero cells

Vero cells were cultured in DMEM medium supplemented with 10% fetal bovine serum (FBS) and 1% penicillin/streptomycin. After culturing, the cells were infected with herpes simplex virus (HSV-1, 0.1 MOI) for 2 hours in DMEM medium supplemented with 1% penicillin/streptomycin. The infection solution was removed, the cells were washed with PBS and then treated with the samples from Preparation example 1 at different concentrations in DMEM supplemented with 10% FBS and 1% penicillin/streptomycin. The cells were subsequently cultured for 18 hours. Afterwards, the cells were recovered, fixed with 4% paraformaldehyde, washed with PBS, and GFP of the virus was quantified using flow cytometry. The cell infection rate was expressed as the infection rate compared to the group (Virus) that had not been treated with any sample.

As a result, as shown in Figure 1, it was found that infection of the herpes simplex virus was suppressed when a treatment was carried out with the extract of grapevine stem or the extract of grapevine main stem bark of the present invention.

In addition, the antiviral effect of the components contained in the grapevine stem extract obtained in Preparation example 2, i.e., vitisin A, ε-viniferin, caraphenol A, betuin, cis-vitisin A, ampelopsin A, lupeol, vitisin B, malibatol A, viniferether B, lupenone, and resveratrol, was also determined by conducting experiments in the same manner as above.

As a result, as shown in Figure 2, at a concentration of 5 µM, vitisin A and vitisin B each showed an inhibition rate of virus replication of 80%, and at a concentration of 10 µM, vitisin A had an inhibition rate of 96%, ε-viniferin had an inhibition rate of 42%, caraphenol A had an inhibition rate of 41%, amphelopsin A had an inhibition rate of 54%, lupeol had an inhibition rate of 59%, and vitisin B had an inhibition rate of 76%. Resveratrol showed 67% inhibition of viral infection.

Therefore, it was found that, among the components contained in the grapevine stem extract, vitisin A and vitisin B have the best antiviral effect.

### Example 2. Antiviral effect of extract of grapevine stem and extract of grapevine main stem bark in SK-N-SH cells

SK-N-SH cells were cultured in DMEM medium supplemented with 10% fetal bovine serum (FBS) and 1% penicillin/streptomycin. After culturing, the cells were infected with herpes simplex virus (HSV-1, 0.1 MOI) for 2 hours in DMEM medium supplemented with 1% penicillin/streptomycin. The infection solution was removed, and the cells were washed with PBS and treated with the samples from Preparation example 1 at different concentrations in DMEM supplemented with 10% FBS and 1% penicillin/streptomycin. The cells were subsequently cultured for 18 hours. Afterwards, the cells were recovered, fixed with 4% paraformaldehyde, washed with PBS, and GFP of the virus was quantified using flow cytometry. The cell infection rate was expressed as the infection rate compared to the group (Virus) that had not been treated with an extract of the grapevine stem or an extract of the grapevine main stem bark

As a result, as shown in Figure 3, it was shown that infection of the herpes simplex virus was suppressed when a treatment was carried out with an ethanol extract of the grapevine stem (fruiting stem), a water extract of the grapevine stem (fruiting stem), a water extract of the grapevine main stem bark, or an ethanol extract of the grapevine main stem bark. It was particularly shown that the infection inhibition effect of the ethanol extract of the grapevine stem (fruiting stem) was significant.

### Example 3. Antiviral effect of grapevine stem extract and its components in U373-MG cells

U373-MG cells, a human-derived glioblastoma, were cultured in RPMI1640 medium supplemented with 10% FBS (Fetal bovine serum) and 1% penicillin/streptomycin. After culturing, the cells were infected with herpes simplex virus (HSV-1, 0.1 MOI) in RPMI1640 medium supplemented with 1% penicillin/streptomycin. After 2 hours, the infection solution was removed. After washing with PBS, the cells were treated for 18 hours with a sample in RPMI1640 supplemented with 10% FBS and 1% penicillin/streptomycin. Afterwards, MTT assay was performed to see whether the cell viability, which showed a decrease by the virus, increases by a sample treatment in U373-MG cells. Cell survival rate was expressed as cell survival rate compared to the untreated group (control).

As a result, as shown in Figure 4, it was shown that when U373-MG cells were treated with the ethanol extract of the grapevine stem (fruiting stem) or the water extract of the grapevine main stem bark, herpes simplex virus infection was suppressed and cell survival rate reduced by virus infection was recovered.

Additionally, as shown in Figure 5A, at a concentration of 10 µM, vitisin A, cis-vitisin A, and vitisin B showed cell survival rates of 87%, 84%, and 76%, which were 2.2-, 2.1-, and 1.8-fold increases compared to the virus-infected group (Virus).

In addition, as a result of separating intracellular proteins and examining the proteins of the herpes simplex virus by Western blot, it was found that at a concentration of 10 µM, vitisin A, cis-vitisin A, and vitisin B reduced gB protein by 99%, 27%, and 66% compared to the virus-infected group (Virus), as shown in Figure 5B. In addition, as a result of checking the inhibition rate of vitisin A against the herpes simplex virus proteins ICP27 and VP16, the inhibition rate was 93% and 100% compared to the virus-infected group (Virus).

### Example 4. Determination of antiviral effect of extract of grapevine stem and extract of grapevine main stem bark in animal model of virus infection

Six-week-old BALB/c female mice were randomly assigned to four groups (10 each), and then intranasally infected with the herpes simplex virus and then orally administered for 7 days with an ethanol extract of grapevine stem (fruiting stem) of 100 mg/kg, water extract of grapevine stem (fruiting stem) of 100 or 300 mg/kg, or water extract of grapevine main stem bark of 300 mg/kg dissolved in sterilized distilled water. The virus-uninfected group (negative control group) and the group that was only infected with the virus without administering any sample (virus-infected group) were administered with sterilized distilled water. Animals showing weight loss of more than 20% compared to the initial weight before the infection were sacrificed and considered death from an ethical point of view.

As a result of checking the body weight and survival rate for 11 days after the virus infection, as shown in Figure 6, the survival rate of the group administered with the grapevine stem extract increased compared to the group infected only with the virus, and the grapevine stem extract of the present invention was able to significantly reduce death caused by the viral infection. In particular, the group administered with the ethanol extract of grapevine stem (fruiting stem) of 100 mg/kg and the water extract of grapevine stem (fruiting stem) of 300 mg/kg showed the best improvement in survival rate.

In addition, as shown in Figure 7, the ethanol extract of the grapevine stem (fruiting stem) was found to inhibit weight loss caused by the virus infection, and from the 9th day of infection, it was found that there was no difference in body weight compared to the negative control group that was not infected with any virus.

In addition, as a result of determining the expression level of viral proteins in mouse brain tissue, it was found that the expression of gB viral protein was significantly reduced in the group administered with the extract of the grapevine stem (fruiting stem) compared to the virus-infected group, as shown in Figure 8. In particular, when the ethanol extract of the grapevine stem (fruiting stem) was administered, the effect of reducing gB viral protein expression was significant.

### Example 5. Determination of change in expression of dementia-related factors, which has been increased by herpes simplex virus injection, by treatment with extract of grapevine stem (fruiting stem)

The experiment was conducted in the same manner as the animal experiment in Example 4 above, but on the 8th day of infection, three mice were randomly selected, brain tissue was collected, and the expression of dementia-related proteins was examined.

As a result, as shown in Figure 9, in the virus-infected group, the expression of amyloid beta (Aβ1-42) increased and the phosphorylation of tau protein (p-tau) was promoted, and the group administered with the extract of the grapevine fruiting stem showed a significantly decreased expression of amyloid beta (Aβ1-42) and phosphorylation of tau protein (p-tau) compared to the virus-infected group.

### Example 6. Determination of change in expression of dementia-related factors, which has been increased by herpes simplex virus injection, by component contained in extract of grapevine stem in U373-MG cells

The experiment was conducted in the same manner as the cell experiment in Example 3, but vitisin A, cis-vitisin A, and vitisin B isolated from grapevine stem extract were treated as test samples, and cell proteins were isolated to determine the expression of proteins that are related to dementia.

As a result, as shown in Figure 10, viral infection increased the expression of amyloid beta (Aβ1-42) and promoted the phosphorylation of tau protein (p-tau), and it was shown that the group treated with vitisin A, a component contained in the grapevine stem extract, significantly reduced the expression of amyloid beta (Aβ1-42) and the phosphorylation of tau protein (p-tau) compared to the virus-infected group.

## Claims

1. A pharmaceutical composition for preventing or treating Alzheimer's disease comprising grapevine stem extract or vitisin A as an effective component.

2. The pharmaceutical composition according to Claim 1, wherein the Alzheimer's disease is Alzheimer's dementia caused by herpes virus infection.

3. The pharmaceutical composition according to Claim 2, wherein the herpes virus is herpes simplex virus type 1 (HSV-1).

4. The pharmaceutical composition according to Claim 1, wherein the vitisin A is isolated from grapevine stem extract.

5. The pharmaceutical composition according to Claim 1, wherein an extract solvent for the grapevine stem extract is water, C₁ to C₄ lower alcohol, or a mixture thereof.

6. The pharmaceutical composition according to Claim 1, wherein the grapevine stem extract or vitisin A inhibits production of amyloid beta and phosphorylation of tau protein.

7. The pharmaceutical composition according to Claim 1, wherein it further comprises a pharmaceutically acceptable carrier, excipient, or diluent in addition to the effective component.

8. A functional health food composition for preventing or ameliorating Alzheimer's disease comprising grapevine stem extract or vitisin A as an effective component.

9. The functional health food composition according to Claim 8, wherein the Alzheimer's disease is Alzheimer's dementia caused by herpes virus infection.

10. The functional health food according to Claim 8, wherein it is prepared in any one formulation selected from powder, granule, pill, tablet, capsule, candy, syrup, and beverage.

11. An animal feed additive for preventing or ameliorating Alzheimer's disease comprising grapevine stem extract or vitisin A as an effective component.
